Europäisches Patentamt

European Patent Office

Office européen des brevets

(19)

(11) Numéro de publication: **0 154 655**
A1

## DEMANDE DE BREVET EUROPEEN

(12)

(21) Numéro de dépôt: 83402300.4

(22) Date de dépôt: 30.11.83

(51) Int. Cl.⁴: **A 61 F 2/14**

(43) Date de publication de la demande: 18.09.85
Bulletin 85/38

(84) Etats contractants désignés: **CH DE GB LI NL SE**

(71) Demandeur: **Meur, Guy, 86 rue Tenbosch, B-1050 Bruxelles (BE)**

(72) Inventeur: **Meur, Guy, 86 rue Tenbosch, B-1050 Bruxelles (BE)**

(74) Mandataire: **Viard, Jean, Cabinet VIARD 28 bis, avenue Mozart, F-75016 Paris (FR)**

(54) Cristallin artificiel.

(57) Cristallin artificiel ou implant intraoculaire comprenant une lentille sur laquelle sont fixées des anses souples.

Selon l'invention, la lentille (1) est disposée dans la chambre postérieure (8) alors que les anses (3) sont conformées pour venir prendre appui, après traversée de la pupille (10) dans l'angle irido-cornéen (15).

Applications: amélioration du maintien de l'implant, la pose étant facilitée.

1

## CRISTALLIN ARTIFICIEL

La présente invention a pour objet un cristallin artificiel ou implant intraoculaire destiné en particulier, mais non exclusivement,à être mis en place après une opération de la cataracte. Le but de cette prothèse est d'éviter à l'opéré le port permanent de lunettes très épaisses destinées à pallier l'absence du cristallin naturel.

Le cristallin naturel chez l'homme est une structure transparente dont le diamètre est de l'ordre de 9mm pour une épaisseur de 5 mm. environ, de forme générale lenticulaire, suspendu derrière l'iris par des fibres zonulaires qui relient le cristallin au corps ciliaire. La cataracte consiste en une opacification de l'une,ou des capsules,antérieure ou postérieure qui constituent le sac cristallin. L'opération de la cataracte consiste en l'ablation du cristallin et est désignée comme "intracapsulaire" lorsque la capsule est retirée en même temps que le cristallin et, comme "extracapsulaire" lorsque la capsule antérieure est extraite avec le cristallin alors que la capsule postérieure est laissée à l'intérieur de l'oeil.

La première lentille intraoculaire a été implantée par RIDLEY en 1949 et, depuis cette date différents types de cristallins artificiels ont été proposés. On peut citer, notamment les brevets US-A- 3 991 426 et US-A- 4 092 743.

Parmi les problèmes que posent l'implantation et le port de lentilles intraoculaires celui de la fixation de l'implant à l'intérieur de la cavité oculaire est l'un des plus critiques. Pour des raisons de tolérance, les lentilles, qui étaient à l'origine disposées dans le plan iridien, sont maintenant implantées soit dans la chambre antérieure constituée par la cornée et l'iris, soit dans la chambre postérieure constituée par l'iris et la membrane hyaloïde de l'humeur vitrée. Les implants sont maintenus par des anses souples prenant appui, dans le cas d'un implant de chambre antérieure, dans l'angle irido-cornéen ou angle de l'oeil et, dans le cas d'un implant de chambre postérieure,

2

dans le sulcus ou corps ciliaire. L'optique est habituellement contituée par une lentille de forme générale plan-convexe en matière synthétique tolérée par l'organisme à partir de laquelle s'étendent, dans une direction sensiblement radiale les haptiques. Celles-ci sont généralement constituées par des anses souples du type "Sinskey" ou "J-loop", dont le matériau peut être le même que celui de la lentille qui, habituellement,présente une convergence de 19 à 21 dioptries.

L'un des inconvénients majeurs de ce type de fixation réside dans les risques de luxation de l'implant lors des mouvements de l'iris. Il devient dangereux de provoquer une dilatation de la pupille en vue de l'observation de la rétine. D'autre part, les contraintes mécaniques qu'imposent les anses aux tissus au niveau des zones de contact provoquent une gêne, voire un traumatisme des tissus qu'il faut traiter par voie médicamenteuse ou opérer. La multiplication des surfaces de contact augmente le risque de formation d'adhérences alors que de faibles surfaces de contact augmentent les risques de luxation de l'implant et les captures iriennes qui consistent dans le passage d'une partie de l'iris en avant ou en arrière de l'implant. Les opérations de suture des anses sur l'iris se sont révélées extrêmement délicates et ne peuvent faire l'objet d'une grande extension.

La présente invention a pour objet de pallier les défauts des implants connus et de faciliter la mise en place et, après opération, le maintien de l'implant.

Selon la présente invention, le cristallin artificiel du type comprenant une optique et des anses souples, l'optique étant implantée dans la chambre postérieure, est caractérisé en ce qu'il comprend au moins deux anses fixées dans la face plane de la lentille plan-convexe, dont la face convexe est tournée vers l'arrière, les anses faisant saillie à partir de la face plane et formant avec celle-ci un angle de l'ordre de 5°, lesdites anses prenant appui, après mise en place, dans l'angle irido-cornéen après traversée de la pupille.

3

Ainsi, l'implant est parfaitement suspendu dans la chambre antérieure alors que l'optique se trouve en chambre postérieure. Tout risque de luxation ou de capture irienne se trouve ainsi supprimé.

De préférence, les anses sont au nombre de trois et leur forme est semicirculaire. Cette disposition permet un auto-centrage de l'implant et assure une bonne stabilité au montage. Par ailleurs, la convexité postérieure de la lentille permet de tendre la capsule postérieure en cas d'ablation extracapsulaire ce qui permet d'éviter dans une large mesure une opacification ultérieure de celle-ci. Toutefois, l' implant selon l'invention peut également être utilisé après une opération intracapsulaire, la convexité postérieure servant dans ce cas à refouler l'hyaloïde du vitré vers l'arrière.

Selon une autre caractéristique de l'invention, la lentille et les anses sont réalisées en polyméthylmétacrylate (PMMA) qui s'est révélé être un matériau convenablement inerte.

D'autres caractéristiques et avantages de l'invention apparaîtront au cours de la description qui va suivre de modes particuliers de réalisation de l'invention, donnés uniquement à titre d'exemples non limitatifs, en regard des dessins qui représentent :
- Les Figs. 1 et 2, respectivement en vue de face et en vue de profil un implant selon l'invention à trois anses;
- Les Figs. 3 et 4, un implant à deux anses;
- La Fig.5, une vue en coupe d'un oeil dans lequel est monté un cristallin artificiel;
- La Fig.6, une vue de face du même oeil.

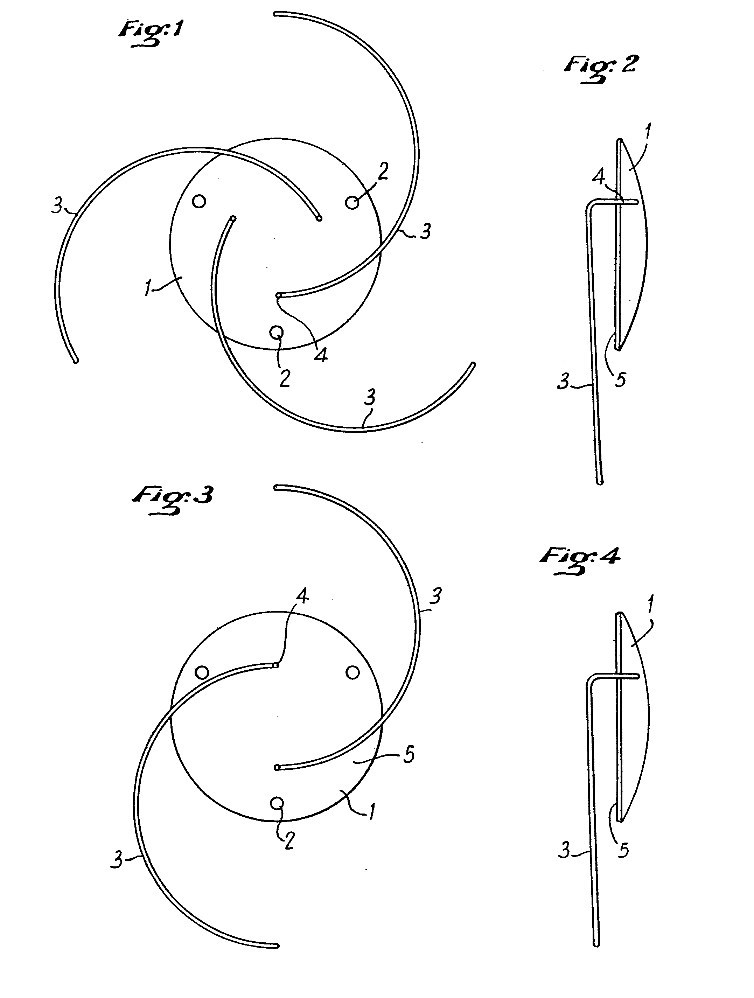

Sur les Figs. 1 et 2, dans la lentille plan convexe 1, sont fixées 3 anses 3 dont la forme est semicirculaire. Cette fixation est obtenue par pénétration, pour chacune des anses, d'un tenon 4 enfoncé dans une direction perpendiculaire à la face plane dans ladite face. La longueur des tenons 4 est telle que l'anse 3 correspondante fasse saillie au-dessus de

0154655

4

de la face plane,le plan de la branche 3 formant avec le tenon 4 un angle inférieur de quelques degré à l'angle droit. Il est en effet essentiel que les anses soient légèrement rabattues par rapport au plan de la lentille pour permettre un bon positionnement de l'implant dans la chambre antérieure.

Le tenon 4 fait lui-même saillie au-dessus de la face plane de la lentille, la longueur de cette saillie permettant au tenon de passer à travers la pupille de l'oeil de sorte que la partie optique de l'implant se trouve en chambre postérieure alors que les haptiques sont en chambre intérieure.

A titre d'exemple, la lentille 1 peut avoir un diamètre de 6 mm., les anses 3 ayant un diamètre de 0,17 mm. L'encombrement hors-tout est de 13,5 mm, le rayon de courbure des anses étant de 4,15 mm. Comme connnu en soi, la lentille 1 présente éventuellement des trous de positionnement 2.

Afin de permettre, d'une part un fonctionnement normal de l'iris et, d'autre part une vision normale du porteur de l'implant, les tenons 4 sont espacés de 2,5 mm.La distance de l'anse 3 au-dessus de la surface plane 5 de la lentille est de 0,8 mm. alors qu'elle n'est plus que de 0,5 mm à l'autre extrêmité de l'anse.

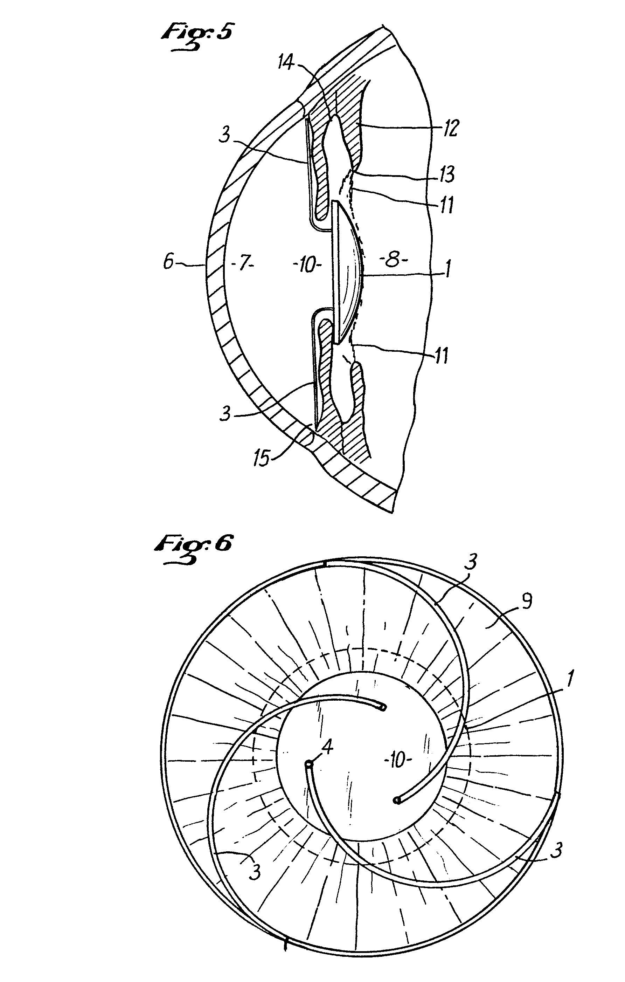

Les Figs 3 et 4 sont identiques aux Figs 1 et 2, à ceci près que le nombre d'anses 3 est de deux au lieu de trois. Le nombre d'anses peut être quelconque sans sortir pour celà du cadre de l'invention.De même, la forme des anses n'est pas nécessairement semicirculaire.

Les Figs 5 et 6 représentent un implant en position à l'intérieur d'un oeil. La Fig.5 est une coupe par un plan vertical alors que la Fig.6 est une vue de face.

Sur la Fig.5, on distingue la cornée transparente 6, la chambre antérieure 7, la pupille 10 au milieu de l'iris 9, la chambre postérieure 8, ainsi qu'en traits pointillés la capsule postérieure 11 réunie à l'extension du corps ciliaire 12 par des fibres zonulaires 13. Alors que dans la technique

5

antérieure les anses des implants de chambre postérieure prenaient appui contre le corps ciliaire 14, conformément à l'invention, les anses 3 prennent appui dans l'angle irido-cornéen 15, après traversée de la pupille 10. Etant donné la position des tenons 4, la pupille peut se contracter ou se dilater librement. D'autre part, en raison de la focalisation introduite par la lentille, le porteur ne voit pas les anses qui, de plus sont transparentes.

La Fig.6 montre la disposition des anses 3 qui, dans l'exemple représenté sont au nombre de trois, au-dessus de l'iris 9, les tenons 4 passant à travers la pupille 10. Comme celà apparaît sur la Fig, les anses 3 viennent reposer dans l'angle 15 d'une manière tangentielle, cette disposition permettant de bénéficier de toutes les qualités élastiques de réaction des anses 3.

Il va de soi que de nombreuses variantes peuvent être apportées, notamment par substitution de moyens techniquement équivalents, sans sortir pour celà du cadre de la présente invention tel qu'il est défini par les revendications annexées.

REVENDICATIONS

1° Cristallin artificiel du type comprenant une lentille à partir de laquelle s'étendent des anses souples, la lentille étant implantée en chambre postérieure, caractérisé en ce qu'il comprend au moins deux anses (3) souples, fixées dans la face plane avant (5) de la lentille (1), les anses faisant saillie à partir de la face (5) et se développant dans un plan incliné d'un angle d'environ cinq degrés sur le plan de la surface (5), les anses (3) prenant appui, après traversée de la pupille (10), dans l'angle irido-cornéen (15) lorsque le cristallin est implanté.

2° Cristallin selon la revendication 1, caractérisé en ce que la forme des anses (3) est une forme semicirculaire.

3° Cristallin artificiel selon l'une des revendications 1 ou 2, caractérisé en ce que le diamètre de la lentille étant de 6 mm., les anses (3) sont fixées dans la face avant par des tenons (4) faisant saillie de 0,8 mm. au dessus de la face (5), la distance entre deux des tenons (4) étant de 2,5 mm.

4° Cristallin artificiel selon l'une quelconque des revendications précédentes, caractérisé en ce que la lentille (1) et les anses (3) sont réalisées en polyméthylmétacrylate.

0154655

1/2

Fig:1

Fig:2

Fig:3

Fig:4

0154655

2/2

*Fig. 5*

*Fig. 6*

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| | | | A 61 F    2/14 |
| X | EP-A-0 053 384  (G.D. FAULKNER)<br>* Abrégé; descriptif; page 7, lignes 26-30; page 11, lignes 3-19; page 2, lignes 8-10; figure 7 * | 1-4 | |
| X | US-A-4 257 130  (J.H. BAYERS)<br>* En entier * | 1-3 | |
| A | US-A-4 340 979  (C.D. KELMAN)<br>* Figures * | 1-4 | |
| A | BE-A- 873 297  (A. GALAND)<br>* Pages 3,4; figures * | 1,3,4 | |
| A | EP-A-0 067 765  (K. HANNA)<br>* Figures 1-3 * | 1,2 | DOMAINES TECHNIQUES RECHERCHES (Int. Cl.4) |
| | ----- | | A 61 F |

Le présent rapport de recherche a été établi pour toutes les revendications

| Lieu de la recherche<br>LA HAYE | Date d'achèvement de la recherche<br>27-07-1984 | Examinateur<br>WOLF C.H.S. |
|---|---|---|